# EUROPEAN PATENT APPLICATION

(11) **EP 1 184 465 A2**
(43) Date of publication of application: **06.03.2002**
(21) Application number: 01306960.4
(22) Date of filing: 16.08.2001
(51) Int. Cl.: C12Q 1/68

(54) **Method for diagnosing polymorphisms in human MCT-1 and drugs related to such polymorphisms**

(30) Priority: 22.08.2000 GB 0020544; 18.09.2000 US 233624
(71) Applicant: AstraZeneca AB, 151 85 Södertälje (SE)
(72) Inventor: Cresswell, Carl John, Macclesfield, Cheshire SK10 4TG (GB); Dudley, Adam Jeston, Wilmington, Delaware 19850-5437 (US)
(74) Representative: Giles, Allen Frank

(57) **Abstract**

This invention relates to polymorphisms in the human MCT-1 gene. The invention also relates to methods and materials for analysing allelic variation in the MCT-1 gene, and to the use of MCT-1 polymorphism in treatment of diseases with MCT-1 transportable drugs.

### SEQUENCE LISTING

<110> AstraZeneca AB

<120> Chemical Compounds

<130> afg

<140>
<141>

<160> 13

<170> PatentIn Ver. 2.1

<210> 1
<211> 15
<212> DNA
<213> Artificial Sequence

<220>
<223> Description of Artificial Sequence:PCR primer

<400> 1
accagcagtt ggagg                                                  15


<210> 2
<211> 15
<212> DNA
<213> Artificial Sequence

<220>
<223> Description of Artificial Sequence:PCR primer

<400> 2
gtttagtagc aagcc                                                  15


<210> 3
<211> 15
<212> DNA
<213> Artificial Sequence

<220>
<223> Description of Artificial Sequence:PCR primer

<400> 3
agaggcgacc attgg                                                  15


<210> 4
<211> 15
<212> DNA
<213> Artificial Sequence

<220>
<223> Description of Artificial Sequence:PCR primer

<400> 4
cccatagatg gtcgg                                                  15


<210> 5
<211> 15
<212> DNA
<213> Artificial Sequence

<220>
<223> Description of Artificial Sequence:PCR primer

<400> 5

attcacccac agagg                                                    15


<210> 6
<211> 15
<212> DNA
<213> Artificial Sequence

<220>
<223> Description of Artificial Sequence:PCR primer

<400> 6
caacatctat actgg                                                    15


<210> 7
<211> 15
<212> DNA
<213> Artificial Sequence

<220>
<223> Description of Artificial Sequence:PCR primer

<400> 7
caatgacatg tatgg                                                    15


<210> 8
<211> 15
<212> DNA
<213> Artificial Sequence

<220>
<223> Description of Artificial Sequence:PCR primer

<400> 8
taaggctctc tagag                                                    15


<210> 9
<211> 15
<212> DNA
<213> Artificial Sequence

<220>
<223> Description of Artificial Sequence:PCR primer

<400> 9
cttaccaata gcctg                                                    15


<210> 10
<211> 15
<212> DNA
<213> Artificial Sequence

<220>
<223> Description of Artificial Sequence:PCR primer

<400> 10
gtgtctagaa cagcc                                                    15


<210> 11
<211> 14
<212> DNA
<213> Artificial Sequence

<220>
<223> Description of Artificial Sequence:PCR primer

<400> 11

agtagatttc tggc                                                    14

<210> 12
<211> 15
<212> DNA
<213> Artificial Sequence

<220>
<223> Description of Artificial Sequence:PCR primer

<400> 12
cacatagcac taagc                                                   15

<210> 13
<211> 2578
<212> DNA
<213> Homo sapiens

<400> 13
tctacactta aaatgccacc agcagttgga ggtccagttg gatacacccc cccagatgga 60
ggctggggct gggcagtggt aattggagct ttcatttcca tcggcttctc ttatgcattt 120
cccaaatcaa ttactgtctt cttcaaagag attgaaggta tattccatgc caccaccagc 180
gaagtgtcat ggatatcctc cataatgttg gctgtcatgt atggtggagg tcctatcagc 240
agtatcctgg tgaataaata tggaagtcgt atagtcatga ttgttggtgg ctgcttgtca 300
ggctgtggct tgattgcagc ttctttctgt aacaccgtac agcaactata cgtctgtatt 360
ggagtcattg gaggtcttgg gcttgccttc aacttgaatc cagctctgac catgattggc 420
aagtatttct acaagaggcg accattggcc aacggactgg ccatggcagg cagccctgtg 480
ttcctctgta ctctggcccc cctcaatcag gttttcttcg gtatctttgg atggagagga 540
agctttctaa ttcttggggg cttgctacta aactgctgtg ttgctggagc cctcatgcga 600
ccaatcgggc ccaagccaac caaggcaggg aaagataagt ctaaagcatc ccttgagaaa 660
gctggaaaat ctggtgtgaa aaaagatctg catgatgcaa atacagatct tattggaaga 720
caccctaaac aagagaaacg atcagtcttc caaacaatta atcagttcct ggacttaacc 780
ctattcaccc acagaggctt tttgctatac ctctctggaa atgtgatcat gtttttggga 840
ctctttgcac ctttggtgtt tcttagtagt tatgggaaga gtcagcatta ttctagtgag 900
aagtctgcct tccttctttc cattctggct tttgttgaca tggtagcccg accatctatg 960
ggacttgtag ccaacacaaa gccaataaga cctcgaattc agtatttctt tgcggcttcc 1020
gttgttgcaa atggagtgtg tcatatgcta gcacctttat ccactaccta tgttggattc 1080
tgtgtctatg cgggattctt tggatttgcc ttcgggtggc tcagctccgt attgtttgaa 1140
acattgatgg accttgttgg accccagagg ttctccagcg ctgtgggatt ggtgaccatt 1200
gtggaatgct gtcctgtcct cctggggcca ccacttttag gtcggctcaa tgacatgtat 1260
ggagactaca aatacacata ctgggcatgt ggcgtcgtcc taattatttc aggtatctat 1320
ctcttcattg gcatgggcat caattatcga cttttggcaa aagaacagaa agcaaacgag 1380
cagaaaaagg aaagtaaaga ggaagagacc agtatagatg ttgctgggaa gccaaatgaa 1440
gttaccaaaa cagcagaatc tccggaccag aaagacacag aaggagggcc caaggaggag 1500
gaaagtccag tctgaatcca tggggctgaa gggtaaattg agcagttcat gacccaggat 1560
atctgaaaat attctactgg cctgtaatct accagtggtg ctcaatgcaa atagtagaca 1620
tttgtgtgga aatcatacca gttgttcatt gatgggattt ttgtttgact ccttaccaat 1680
agcctgaatt tgaggaggga atgattggta gcaaaggatg ggggaaagaa gtaggttctg 1740
ttttgttttg ttttaatctt agcttttaat agtgtcataa agattataat atgtgcctta 1800
agttttagtc tttagaactc tagagagcct taacttctta aaccattttt gctgaattca 1860
tctatttcga gtgttgtgtt aaaaggaaaa ataacaacta acttgtttga ggcaaatcta 1920
aaatttaaaa ttaatcttgc ttcattgtta catgtaatat atttcagaca ttttcactgg 1980
aagatttatg aacagaaata ttggttgaaa gttagagatt ttacaaaatg ctgacaaaaa 2040
tattttccta gcatcagtag atttctggca tatgtttctg ctagctatat atttaggaaa 2100
ttcaaagcat aaaactttgg caacatcttg gctgttctag acacagtgta cttgtcaacc 2160
cctctcaggt accttttctt gggatgctta ttagaagcca agtaaagtgc ttaaggtttg 2220
ttttcattaa attagctatt tctgctcccc tgttcaaaga tgcattttga gtgtttatag 2280
atcactgccc tttttgaaat cacctggtat tatttttctt actggaaaag ttagtattaa 2340
aatctacaga actacatatt tgtgcctcct tggtaaatac aacacatcta attaaatgta 2400
gacagatatt tcaaacatca gctgaattca cttaagtttt tccaaaacct cagttaaact 2460
gtgaagctat tggaattttt ttttcctgga atttttcccc tttgattcac agtggtccca 2520
tttatatctg cttctagctt agtgctatgt gtgagatatg tgtgtgtttg gtgtttt   2578

<210> 14
<211> 500
<212> PRT
<213> Homo sapiens

<400> 14
Met Pro Pro Ala Val Gly Gly Pro Val Gly Tyr Thr Pro Pro Asp Gly
1               5                   10                  15

Gly Trp Gly Trp Ala Val Val Ile Gly Ala Phe Ile Ser Ile Gly Phe
            20                  25                  30

Ser Tyr Ala Phe Pro Lys Ser Ile Thr Val Phe Phe Lys Glu Ile Glu
        35                  40                  45

Gly Ile Phe His Ala Thr Thr Ser Glu Val Ser Trp Ile Ser Ser Ile
    50                  55                  60

Met Leu Ala Val Met Tyr Gly Gly Gly Pro Ile Ser Ser Ile Leu Val
65                  70                  75                  80

Asn Lys Tyr Gly Ser Arg Ile Val Met Ile Val Gly Gly Cys Leu Ser
                85                  90                  95

Gly Cys Gly Leu Ile Ala Ala Ser Phe Cys Asn Thr Val Gln Gln Leu
            100                 105                 110

Tyr Val Cys Ile Gly Val Ile Gly Gly Leu Gly Leu Ala Phe Asn Leu
        115                 120                 125

Asn Pro Ala Leu Thr Met Ile Gly Lys Tyr Phe Tyr Lys Arg Arg Pro
    130                 135                 140

Leu Ala Asn Gly Leu Ala Met Ala Gly Ser Pro Val Phe Leu Cys Thr
145                 150                 155                 160

Leu Ala Pro Leu Asn Gln Val Phe Phe Gly Ile Phe Gly Trp Arg Gly
                165                 170                 175

Ser Phe Leu Ile Leu Gly Gly Leu Leu Leu Asn Cys Cys Val Ala Gly
            180                 185                 190

Ala Leu Met Arg Pro Ile Gly Pro Lys Pro Thr Lys Ala Gly Lys Asp
        195                 200                 205

Lys Ser Lys Ala Ser Leu Glu Lys Ala Gly Lys Ser Gly Val Lys Lys
    210                 215                 220

Asp Leu His Asp Ala Asn Thr Asp Leu Ile Gly Arg His Pro Lys Gln
225                 230                 235                 240

Glu Lys Arg Ser Val Phe Gln Thr Ile Asn Gln Phe Leu Asp Leu Thr
                245                 250                 255

Leu Phe Thr His Arg Gly Phe Leu Leu Tyr Leu Ser Gly Asn Val Ile
            260                 265                 270

Met Phe Phe Gly Leu Phe Ala Pro Leu Val Phe Leu Ser Ser Tyr Gly
        275                 280                 285

Lys Ser Gln His Tyr Ser Ser Glu Lys Ser Ala Phe Leu Leu Ser Ile
    290                 295                 300

Leu Ala Phe Val Asp Met Val Ala Arg Pro Ser Met Gly Leu Val Ala
305                 310                 315                 320

Asn Thr Lys Pro Ile Arg Pro Arg Ile Gln Tyr Phe Phe Ala Ala Ser
                325                 330                 335

Val Val Ala Asn Gly Val Cys His Met Leu Ala Pro Leu Ser Thr Thr
            340                 345                 350

Tyr Val Gly Phe Cys Val Tyr Ala Gly Phe Phe Gly Phe Ala Phe Gly
        355                 360                 365

Trp Leu Ser Ser Val Leu Phe Glu Thr Leu Met Asp Leu Val Gly Pro
    370                 375                 380

Gln Arg Phe Ser Ser Ala Val Gly Leu Val Thr Ile Val Glu Cys Cys
385                 390                 395                 400

Pro Val Leu Leu Gly Pro Pro Leu Leu Gly Arg Leu Asn Asp Met Tyr
                405                 410                 415

Gly Asp Tyr Lys Tyr Thr Tyr Trp Ala Cys Gly Val Val Leu Ile Ile
            420                 425                 430

Ser Gly Ile Tyr Leu Phe Ile Gly Met Gly Ile Asn Tyr Arg Leu Leu
        435                 440                 445

Ala Lys Glu Gln Lys Ala Asn Glu Gln Lys Lys Glu Ser Lys Glu Glu
    450                 455                 460

Glu Thr Ser Ile Asp Val Ala Gly Lys Pro Asn Glu Val Thr Lys Thr
465                 470                 475                 480

Ala Glu Ser Pro Asp Gln Lys Asp Thr Glu Gly Gly Pro Lys Glu Glu
                485                 490                 495

Glu Ser Pro Val
            500

<210> 15
<211> 22
<212> DNA
<213> Artificial Sequence

<220>
<223> Description of Artificial Sequence:PCR primer

<400> 15
ctccggacca gaaagacaca gc                                          22


<210> 16
<211> 20
<212> DNA
<213> Artificial Sequence

<400> 16
gaagttaagg ctctctagag                                             20

## Description

This invention relates to polymorphisms in the human MCT- 1 gene and corresponding novel allelic polypeptides encoded thereby. The invention also relates to methods and materials for analysing allelic variation in the MCT-1 gene, and to the use of MCT-1 polymorphism in treatment of diseases with MCT-1 transportable drugs.

Monocarboxylic acids play a major role in the metabolism of all cells, with lactic acid, the end product of glycolysis, being especially important. Some tissues, such as white skeletal muscle, red blood cells and many tumour cells, rely on this pathway to produce most of their ATP under normal physiological conditions, while all tissues become dependent on this pathway during hypoxia or ischaemia. Glycolysis produces two molecules of lactic acid for every glucose molecule consumed, and these must be transported out of the cell if high rates of glycolysis are to be maintained. If efflux of lactic acid from the cell does not keep pace with production, intracellular concentrations increase and cause the pH of the cytosol to decrease. This leads to inhibition of phosphofructokinase and hence glycolysis. Other tissues, such as brain, heart and red skeletal muscle, readily oxidize lactic acid, which may become a major respiratory fuel under some conditions. In these tissues lactic acid must be rapidly transported into the cell. The same is true for tissues such as the liver, which, through the operation of the Cori cycle, utilise lactate as their dominant gluconeogenic substrate (Denton, R. M. and Halestrap, A. P. (1979) Essays Biochem. 15, 37-47: Juel, C. (1997) Physiol. Rev. 77, 321-358.) Although it is lactic acid that is both produced and utilized by metabolism, the pK of lactic acid is 3.86, which ensures that it dissociates almost entirely to the lactate anion at physiological pH. This charged species cannot cross the plasma membrane by free diffusion, but requires a specific transport mechanism, provided by proton-linked monocarboxylate transporters (MCTs). These transporters catalyse the facilitated diffusion of lactate with a proton. There is no energy input other than that provided by the concentration gradients of lactate and protons, although the latter, in the form of a pH gradient, can drive the accumulation or exclusion of the lactate anion (Poole, R. C. and Halestrap, A. P. (1993) Am. J. Physiol. 264, C761-C782.: Juel, C. (1997) Physiol. Rev. 77, 321-358). A polymorphism Glu490Asp in MCT1 has been described by Merezhinskaya (2000), Muscle and Nerve, 23, 90-97.

MCT-1 is thought to be involved in the transport of drugs involved in lipid lowering e.g. statins. Statins have been referred to as a first-line therapy for patients with atherosclerotic vascular diseases.

All positions herein of polymorphisms in the MCT-1 polynucleotide relate to the position in SEQ ID NO 13 unless stated otherwise or apparent from the context.

All positions herein of polymorphisms in the MCT-1 polypeptide relate to the position in SEQ ID NO 14 unless stated otherwise or apparent from the context.

One approach is to use knowledge of polymorphisms to help identify patients most suited to therapy with particular pharmaceutical agents (this is often termed "pharmacogenetics"). Pharmacogenetics can also be used in pharmaceutical research to assist the drug selection process. Polymorphisms are used in mapping the human genome and to elucidate the genetic component of diseases. The reader is directed to the following references for background details on pharmacogenetics and other uses of polymorphism detection: Linder *et al.* (1997), Clinical Chemistry, **43,** 254; Marshall (1997), Nature Biotechnology, **15,** 1249; International Patent Application WO 97/40462, Spectra Biomedical; and Schafer *et al.* (1998), Nature Biotechnology, **16,** 33.

Clinical trials have shown that patient response to treatment with pharmaceuticals is often heterogeneous. Thus there is a need for improved approaches to pharmaceutical agent design and therapy.

Point mutations in polypeptides will be referred to as follows: natural amino acid (using 1 or 3 letter nomenclature) , position, new amino acid. For (a hypothetical) example "D25K" or "Asp25Lys" means that at position 25 an aspartic acid (D) has been changed to lysine (K). Multiple mutations in one polypeptide will be shown between square brackets with individual mutations separated by commas.

The present invention is based on the discovery of polymorphisms in MCT-1. In particular, we have found two single nucleotide polymorphisms (SNPs) in the MCT-1 gene.

According to one aspect of the present invention there is provided a method for the diagnosis of a polymorphism in MCT-1 in a human, which method comprises determining the sequence of the nucleic acid of the human at at least one polymorphic position selected from one or more of the following positions:
positions 1450 and 2461 in the polynucleotide sequence of the MCT-1 gene as defined by the position in SEQ ID NO: 13.

The term human includes both a human having or suspected of having a MCT- 1 mediated disease and an asymptomatic human who may be tested for predisposition or susceptibility to such disease. At each position the human may be homozygous for an allele or the human may be a heterozygote.

In one embodiment of the invention preferably the method for diagnosis described herein is one in which the single nucleotide polymorphism at position 1450 is presence of A and/or G.

In another embodiment of the invention preferably the method for diagnosis described herein is one in which the single nucleotide polymorphism at position 2461 is the presence of A and/or G.

The method for diagnosis is preferably one in which the sequence is determined by a method selected from amplification refractory mutation system and restriction fragment length polymorphism.

The test sample of nucleic acid is conveniently a sample of blood, bronchoalveolar lavage fluid, sputum, or other body fluid or tissue obtained from an individual. It will be appreciated that the test sample may equally be a nucleic acid sequence corresponding to the sequence in the test sample, that is to say that all or a part of the region in the sample nucleic acid may firstly be amplified using any convenient technique e.g. PCR, before analysis of allelic variation.

It will be apparent to the person skilled in the art that there are a large number of analytical procedures which may be used to detect the presence or absence of variant nucleotides at one or more polymorphic positions of the invention. In general, the detection of allelic variation requires a mutation discrimination technique, optionally an amplification reaction and optionally a signal generation system. Table 1 lists a number of mutation detection techniques, some based on the PCR. These may be used in combination with a number of signal generation systems, a selection of which is listed in Table 2. Further amplification techniques are listed in Table 3. Many current methods for the detection of allelic variation are reviewed by Nollau *et al*., Clin. Chem. **43,** 1114-1120, 1997; and in standard textbooks, for example "Laboratory Protocols for Mutation Detection", Ed. by U. Landegren, Oxford University Press, 1996 and "PCR", 2^{nd} Edition by Newton & Graham, BIOS Scientific Publishers Limited, 1997.

| **Abbreviations:** | |
|---|---|
| ALEX™ | Amplification refractory mutation system linear extension |
| APEX | Arrayed primer extension |
| ARMS™ | Amplification refractory mutation system |
| b-DNA | Branched DNA |
| bp | base pair |
| CMC | Chemical mismatch cleavage |
| COPS | Competitive oligonucleotide priming system |
| DGGE | Denaturing gradient gel electrophoresis |
| FRET | Fluorescence resonance energy transfer |
| HMG-CoA | 3-hydroxy-3-methylglutaryl-coenzyme A |
| LCR | Ligase chain reaction |
| MASDA | Multiple allele specific diagnostic assay |
| MCT-1 | monocarboxylate transporter, human |
| NASBA | Nucleic acid sequence based amplification |
| OLA | Oligonucleotide ligation assay |
| PCR | Polymerase chain reaction |
| PTT | Protein truncation test |
| RFLP | Restriction fragment length polymorphism |
| SDA | Strand displacement amplification |
| SNP | Single nucleotide polymorphism |
| SSCP | Single-strand conformation polymorphism analysis |
| SSR | Self sustained replication |
| TGGE | Temperature gradient gel electrophoresis |

**Table 1 -**

| Mutation Detection Techniques |
|---|
| **General:** DNA sequencing, Sequencing by hybridisation |
| **Scanning:** PTT∗, SSCP, DGGE, TGGE, Cleavase, Heteroduplex analysis, CMC, Enzymatic mismatch cleavage |
| **Hybridisation Based** |
| Solid phase hybridisation: Dot blots, MASDA, Reverse dot blots, Oligonucleotide arrays (DNA Chips) |
| Solution phase hybridisation: Taqman™ - US-5210015 & US-5487972 (Hoffmann-La Roche), Molecular Beacons - Tyagi *et al* (1996), Nature Biotechnology, **14**, 303; WO 95/13399 (Public Health Inst., New York) |
| **Extension Based:** ARMS™, ALEX™ - European Patent No. EP 332435 B1 (Zeneca Limited), COPS - Gibbs *et al* (1989), Nucleic Acids Research, **17,** 2347. |
| **Incorporation Based:** Mini-sequencing, APEX |
| **Restriction Enzyme Based:** RFLP, Restriction site generating PCR |
| **Ligation Based:** OLA |
| **Other:** Invader assay |

| |
|---|
| ∗ Note: not useful for detection of promoter polymorphisms. |

**Table 2 -**

| Signal Generation or Detection Systems |
|---|
| **Fluorescence:** FRET, Fluorescence quenching, Fluorescence polarisation - United Kingdom Patent No. 2228998 (Zeneca Limited) |
| **Other:** Chemiluminescence, Electrochemiluminescence, Raman, Radioactivity, Colorimetric, Hybridisation protection assay, Mass spectrometry |

**Table 3 -**

| Further Amplification Methods |
|---|
| SSR, NASBA, LCR, SDA, b-DNA |

Preferred mutation detection techniques include ARMS™, ALEX™, COPS, Taqman, Molecular Beacons, RFLP, and restriction site based PCR and FRET techniques.

Particularly preferred methods include ARMS™ and RFLP based methods. ARMS™ is an especially preferred method.

In a further aspect, the diagnostic methods of the invention are used to assess the pharmacogenetics of a drug transportable by MCT-1.

Assays, for example reporter-based assays, may be devised to detect whether one or more of the above polymorphisms affect transcription levels and/or message stability.

Individuals who carry particular allelic variants of the MCT-1 gene may therefore exhibit differences in their ability to regulate protein biosynthesis under different physiological conditions and will display altered abilities to react to different diseases. In addition, differences arising as a result of allelic variation may have a direct effect on the response of an individual to drug therapy. The diagnostic methods of the invention may be useful both to predict the clinical response to such agents and to determine therapeutic dose.

In a further aspect, the diagnostic methods of the invention, are used to assess the predisposition and/or susceptibility of an individual to diseases mediated by MCT-1. This may be particularly relevant in the development of hyperlipoproteinemia and cardiovascular disease and the present invention may be used to recognise individuals who are particularly at risk from developing these conditions.

In a further aspect, the diagnostic methods of the invention are used in the development of new drug therapies which selectively target one or more allelic variants of the MCT-1 gene. Identification of a link between a particular allelic variant and predisposition to disease development or response to drug therapy may have a significant impact on the design of new drugs. Drugs may be designed to regulate the biological activity of variants implicated in the disease process whilst minimising effects on other variants.

In a further diagnostic aspect of the invention the presence or absence of variant nucleotides is detected by reference to the loss or gain of, optionally engineered, sites recognised by restriction enzymes.

According to another aspect of the present invention there is provided a human MCT-1 gene or its complementary strand comprising a variant allelic polymorphism at one or more of positions defined herein or a fragment thereof of at least 20 bases comprising at least one novel polymorphism.

Fragments are at least 17 bases, more preferably at least 20 bases, more preferably at least 30 bases.

According to another aspect of the present invention there is provided a polynucleotide comprising at least 20 bases of the human MCT-1 gene and comprising an allelic variant selected from any one of the following:

| **variant** | **Position in SEQ ID NO 13** |
|---|---|
| G | 1450 |
| G | 2461 |

According to another aspect of the present invention there is provided a human MCT-1 gene or its complementary strand comprising an allelic variant, preferably corresponding with one or more the positions defined herein or a fragment thereof of at least 20 bases comprising at least one allelic variant.

Fragments are at least 17 bases, more preferably at least 20 bases, more preferably at least 30 bases.

The invention further provides a nucleotide primer which can detect a polymorphism of the invention.

According to another aspect of the present invention there is provided an allele specific primer capable of detecting a MCT-1 gene polymorphism, preferably at one or more of the positions as defined herein.

An allele specific primer is used, generally together with a constant primer, in an amplification reaction such as a PCR reaction, which provides the discrimination between alleles through selective amplification of one allele at a particular sequence position e.g. as used for APMS™ assays. The allele specific primer is preferably 17-50 nucleotides, more preferably about 17-35 nucleotides, more preferably about 17-30 nucleotides.

An allele specific primer preferably corresponds exactly with the allele to be detected but derivatives thereof are also contemplated wherein about 6-8 of the nucleotides at the 3' terminus correspond with the allele to be detected and wherein up to 10, such as up to 8, 6, 4, 2, or 1 of the remaining nucleotides may be varied without significantly affecting the properties of the primer.

Primers may be manufactured using any convenient method of synthesis. Examples of such methods may be found in standard textbooks, for example "Protocols for Oligonucleotides and Analogues; Synthesis and Properties," Methods in Molecular Biology Series; Volume 20; Ed. Sudhir Agrawal, Humana ISBN: 0-89603-247-7; 1993; 1^{st} Edition. If required the primer(s) may be labelled to facilitate detection.

According to another aspect of the present invention there is provided an allele-specific oligonucleotide probe capable of detecting a MCT-1 gene polymorphism, preferably at one or more of the positions defined herein.

The allele-specific oligonucleotide probe is preferably 17-50 nucleotides, more preferably about 17-35 nucleotides, more preferably about 17-30 nucleotides.

The design of such probes will be apparent to the molecular biologist of ordinary skill. Such probes are of any convenient length such as up to 50 bases, up to 40 bases, more conveniently up to 30 bases in length, such as for example 8-25 or 8-15 bases in length. In general such probes will comprise base sequences entirely complementary to the corresponding wild type or variant locus in the gene. However, if required one or more mismatches may be introduced, provided that the discriminatory power of the oligonucleotide probe is not unduly affected. The probes of the invention may carry one or more labels to facilitate detection.

According to another aspect of the present invention there is provided an allele specific primer or an allele specific oligonucleotide probe capable of detecting a MCT- 1 gene polymorphism at one of the positions defined herein.

According to another aspect of the present invention there is provided a diagnostic kit comprising an allele specific oligonucleotide probe of the invention and/or an allele-specific primer of the invention.

The diagnostic kits may comprise appropriate packaging and instructions for use in the methods of the invention. Such kits may further comprise appropriate buffer(s) and polymerase(s) such as thermostable polymerases, for example taq polymerase.

In another aspect of the invention, the single nucleotide polymorphisms of this invention may be used as genetic markers in linkage studies. This particularly applies to the polymorphisms of relatively high frequency. The MCT-1 gene is on chromosome chromosome 1p13.2-p12. Low frequency polymorphisms may be particularly useful for haplotyping as described below. A haplotype is a set of alleles found at linked polymorphic sites (such as within a gene) on a single (paternal or maternal) chromosome. If recombination within the gene is random, there may be as many as 2n haplotypes, where 2 is the number of alleles at each SNP and n is the number of SNPs. One approach to identifying mutations or polymorphisms which are correlated with clinical response is to carry out an association study using all the haplotypes that can be identified in the population of interest. The frequency of each haplotype is limited by the frequency of its rarest allele, so that SNPs with low frequency alleles are particularly useful as markers of low frequency haplotypes. As particular mutations or polymorphisms associated with certain clinical features, such as adverse or abnormal events, are likely to be of low frequency within the population, low frequency SNPs may be particularly useful in identifying these mutations (for examples see: Linkage disequilibrium at the cystathionine beta synthase (CBS) locus and the association between genetic variation at the CBS locus and plasma levels of homocysteine. *Ann Hum Genet* (1998) 62:481-90, De Stefano V, Dekou V, Nicaud V, Chasse JF, London J, Stansbie D, Humphries SE, and Gudnason V; and Variation at the von willebrand factor (vWF) gene locus is associated with plasma vWF:Ag levels: identification of three novel single nucleotide polymorphisms in the vWF gene promoter. *Blood* (1999) 93:4277-83, Keightley AM, Lam YM, Brady JN, Cameron CL, Lillicrap D).

According to another aspect of the present invention there is provided a computer readable medium comprising at least one novel sequence of the invention stored on the medium. The computer readable medium may be used, for example, in homology searching, mapping, haplotyping, genotyping or pharmacogenetic analysis.

According to another aspect of the present invention there is provided a method of treating a human in need of treatment with a drug transportable by MCT- 1 in which the method comprises:
i) diagnosis of a polymorphism in MCT-1 in the human, which diagnosis comprises determining the sequence of the human at one or more of the following positions:
   positions 1450, 1482 and 2461 in the sequence of the MCT-1 polynucleotide as defined by the position in SEQ ID NO: 13; and
   at position 490 of human MCT-1 polypeptide as defined by the position in SEQ ID NO 14; and
ii) administering an effective amount of the drug.

Preferably determination of the status of the human is clinically useful. Examples of clinical usefulness include deciding which statin drug or drugs to administer and/or in deciding on the effective amount of the statin drug or drugs. Statins already approved for use in humans include atorvastatin, cerivastatin, fluvastatin, pravastatin and simvastatin. The reader is referred to the following references for further information: Drugs and Therapy Perspectives (12^{th} May 1997), **9**: 1-6; Chong (1997) Pharmacotherapy **17**: 1157-1177; Kellick (1997) Formulary **32**: 352; Kathawala (1991) Medicinal Research Reviews, **11**: 121-146; Jahng (1995) Drugs of the Future **20**: 387-404, and Current Opinion in Lipidology, (1997), **8**, 362 - 368. A preferred statin drug is compound 3a (S-4522) in Watanabe (1997) Bioorganic and Medicinal Chemistry **5:** 437-444, presently known as rosuvastatin (Olsson, 2001, The American Journal of Cardiology, **87,** 33-36). The term "drug transportable by MCT-1" means that transport by MCT-1 in humans is an important part of a drug exerting its pharmceutical effect in man. For example, some statins have to be transported to the liver by MCT-1 to exert their lipid lowering effects.

According to another aspect of the present invention there is provided use of a drug transportable by MCT-1 in preparation of a medicament for treating a disease in a human diagnosed as having a MCT1 polymorphism therein, preferably at one or more of the positions defined herein. Preferably the disease is cardiovascular. Preferably the medicament is a statin, most preferably rosuvastatin.

According to another aspect of the present invention there is provided a pharmaceutical pack comprising a MCT-1 transportable drug and instructions for administration of the drug to humans diagnostically tested for a single nucleotide polymorphism therein, preferably at one or more of the positions defined herein.

The invention will now be illustrated but not limited by reference to the following Examples. All temperatures are in degrees Celsius.

In the Examples below, unless otherwise stated, the following methodology and materials have been applied.

AMPLITAQ™ ,available from Perkin-Elmer Cetus, is used as the source of thermostable DNA polymerase.

General molecular biology procedures can be followed from any of the methods described in "Molecular Cloning - A Laboratory Manual" Second Edition, Sambrook, Fritsch and Maniatis (Cold Spring Harbor Laboratory, 1989).

Electropherograms were obtained in a standard manner: data was collected by ABI377 data collection software and the wave form generated by ABI Prism sequencing analysis (2.1.2).

### Example 1

### Identification of Polymorphisms

### 1. Methods

### DNA Preparation

DNA was prepared from frozen blood samples collected in EDTA following protocol I (Molecular Cloning: A Laboratory Manual, p392, Sambrook, Fritsch and Maniatis, 2^{nd} Edition, Cold Spring Harbor Press, 1989) with the following modifications. The thawed blood was diluted in an equal volume of standard saline citrate instead of phosphate buffered saline to remove lysed red blood cells. Samples were extracted with phenol, then phenol/chloroform and then chloroform rather than with three phenol extractions. The DNA was dissolved in deionised water.

### Template Preparation

Templates were prepared by PCR using the oligonucleotide primers and annealing temperatures set out below. The extension temperature was 72° and denaturation temperature 94°. Generally 50 ng of genomic DNA was used in each reaction and subjected to 35 cycles of PCR. Where described below, the primary fragment was diluted 1/100 and two microlitres were used as template for amplification of secondary fragments. PCR was performed in two stages (primary fragment then secondary fragment) to ensure specific amplification of the desired target sequence.

| Positions relate to SEQ ID NO 13 | | | | |
|---|---|---|---|---|
| Fragment | Forward Oligo | Reverse Oligo | Annealing Temp | Time |
| 13-578 | 13-32 | 559-578 | 58°C | 60s |
| 429-967 | 429-448 | 948-967 | 58°C | 60s |
| 778-1428 | 778-797 | 1409-1428 | 58°C | 60s |
| 1243-1837 | 1243-1262 | 1818-1837 | 58°C | 60s |
| 1667-2149 | 1667-1786 | 2130-2149 | 58°C | 60s |
| 2051-2556 | 2051-2070 | 2537-2556 | 58°C | 60s |

For dye-primer sequencing these primers were modified to include M13 forward and reverse primer sequences (ABI protocol P/N 402114, Applied Biosystems) at the 5' end of the forward and reverse oligonucleotides respectively.

| MCT1 oligos for cDNA Amplification | |
|---|---|
| Product 1F (13-32) | SEQ ID NO 1 |
| Product 1R (559-578) | SEQ ID NO 2 |
| Product 2F (429-448) | SEQ ID NO 3 |
| Product 2R (948-967) | SEQ ID NO 4 |
| Product 3F (778-797) | SEQ ID NO 5 |
| Product 3R (1428-1409) | SEQ ID NO 6 |
| Product 4F (1243-1262) | SEQ ID NO 7 |
| Product 4R (1837-1818) | SEQ ID NO 8 |
| Product 5F (1667-1786) | SEQ ID NO 9 |
| Product 5R (2149-2130) | SEQ ID NO 10 |
| Product 6F (2051-2070) | SEQ ID NO 11 |
| Product 6R (2556-2537) | SEQ ID NO 12 |

| | |
|---|---|
| F= forward, | |
| R= reverse | |

### Dye Primer Sequencing

Dye-primer sequencing using M13 forward and reverse primers was as described in the ABI protocol P/N 402114 for the ABI Prism^{tm} dye primer cycle sequencing core kit with "AmpliTaq FS" DNA polymerase, modified in that the annealing temperature was 45 °C and DMSO was added to the cycle sequencing mix to a final concentration of 5%.

The extension reactions for each base were pooled, ethanol/sodium acetate precipitated, washed and resuspended in formamide loading buffer.

4.25% Acrylamide gels were run on an automated sequencer (ABI 377, Applied Biosystems).

### Results

### Polymorphisms

| **Position** | **polymorphism** | **frequency** |
|---|---|---|
| 1450 | A→G | 98 % G |
| 1482 | A→T (Glu490Asp) | 46.7% A, 53.3% T |
| 2461 | A→G | 97.8% G, 2.2% A |

The allele frequencies were based on analysis of 23 individuals. The polymorphism Glu490Asp has been described by Merezhinskaya (2000), Muscle and Nerve, 23, 90-97.

### Example 2

### Diagnostic assay by engineered RFLP for polymorphism at position 1482 of MCT-1 Methods (DNA and template preparation as in Example 1)

| | | |
|---|---|---|
| Diagnostic Primer | 1460-1481 | SEQ ID NO 15 |
| Constant Primer | 1818-1837 | SEQ ID NO 16 |

The diagnostic primer contains a single mismatch from the wild type sequence at the 3' residue (A→C).

PCR amplification using these primers will generate a product of 377 bp. The use of the diagnostic primer on a template creates a PvuII recognition sequence at the site of the polymorphism. PvuII (New England Biolabs) can therefore distinguish the two polymorphic variants.

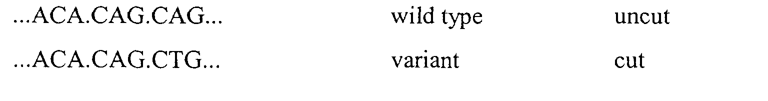

## Claims

1. A method for the diagnosis of a polymorphism in MCT-1 in a human, which method comprises determining the sequence of the nucleic acid of the human at at least one polymorphic position selected from one or more of the following positions: positions 1450 and 2461 in the polynucleotide sequence of the MCT-1 gene as defined by the position in SEQ ID NO: 13.

2. A method according to claim one in which the polymorphisms are further defined as follows:
at position 1450 is presence of A and/or G; and
at position 2461 is the presence of A and/or G.

3. A method for diagnosis according to claim 1 or 2 in which the sequence is determined by a method selected from amplification refractory mutation system and restriction fragment length polymorphism.

4. A polynucleotide comprising at least 20 bases of the human MCT-1 gene and comprising an allelic variant selected from any one of the following:
| variant | Position in SEQ ID NO 13 |
|---|---|
| G | 1450 |
| G | 2461 |

5. An allele specific primer capable of detecting a MCT-1 gene polymorphism at one or more of the positions as defined in claim 1.

6. An allele-specific oligonucleotide probe capable of detecting a MCT-1 gene polymorphism at one or more of the positions defined in claim 1.

7. A diagnostic kit comprising an allele specific oligonucleotide probe as defined in claim 6 and/or an allele-specific primer as defined in claim 5.

8. Use of a polymorphism as defined in claim 1 as a genetic marker in linkage studies.

9. A computer readable medium comprising at least one variant sequence as defined in claim 4 stored on the medium.

10. Use of a drug transportable by MCT-1 in preparation of a medicament for treating a disease in a human diagnosed as having a MCT-1 polymorphism at one or more of the following positions:
positions 1450, 1482 and 2461 in the sequence of the MCT-1 polynucleotide as defined by the position in SEQ ID NO: 13; and
at position 490 of human MCT-1 polypeptide as defined by the position in SEQ ID NO 14.

11. A use according to claim 10 in which the drug is a statin.

12. A use according to claim 10 in which the drug is rosuvastatin.
